# EUROPEAN PATENT SPECIFICATION

(11) **EP 2 521 731 B1**
(45) Date of publication and mention of the grant of the patent: **02.04.2014**
(21) Application number: 11705998.0
(22) Date of filing: 04.01.2011
(51) Int. Cl.: C07K 5/02, A61K 31/198

(54) **COMPOUNDS WITH BOTH ANALGESIC AND ANTI-HYPERALGESIC EFFICACY**
VERBINDUNGEN MIT ANALGETISCHER UND ANTI-HYPERALGETISCHER WIRKUNG
COMPOSÉS AYANT UNE EFFICACITÉ À LA FOIS ANALGÉSIQUE ET ANTIHYPERALGÉSIQUE

(30) Priority: 04.01.2010 IT FI20100001
(43) Date of publication of application: 14.11.2012
(73) Proprietor: Consorzio Interuniversitario Nazionale Per La Scienza E La Tecnologia Dei Materiali (INSTM), 50121 Firenze (IT); Nativi, Cristina, 50134 Firenze (IT); Ghelardini, Carla, 51100 Pistoia (IT); La Marca, Giancarlo, 50125 Firenze (IT); Dragoni, Elisa, 52010 Arezzo (IT)
(72) Inventor: NATIVI, Cristina, I-50134 Firenze (IT); GHELARDINI, Carla, I-51100 Pistoia (IT); LA MARCA, Giancarlo, I-50125 Firenze (IT); DRAGONI, Elisa, I-52010 Arezzo (IT)
(74) Representative: Valenza, Silvia
(86) International application number: PCT/IB2011/050018
(87) International publication number: WO 2011/080725

(56) References cited:
- EP-A1- 1 297 830
- US-A1- 2006 216 251

## Description

### Field of the invention

The present invention relates to the field of organic compounds containing heterocycles having pharmacological efficacy as analgesics.

### State of the art

The World Health Organisation (W.H.O.) defines neuropathic pain as: "An unpleasant sensation and a negative-affective emotional experience, associated with actual or potential tissue damage, or described in terms of such damage". The International Association for the Study of Pain (IASP) defines it as: "An unpleasant sensory and emotional experience associated with actual or potential tissue damage, or described as such".

Neuropathic pain is a significant problem in neurology in that it occurs frequently and is often disabling due to its irritating and chronic character.

Examples thereof are: post-herpetic pain, phantom limb pain which can arise after an amputation, pain present in peripheral neuropathy such as with diabetes or AIDS, so-called complex regional pain syndrome or reflex sympathetic dystrophy pain, and pain from lesions of the central nervous system. These latter can be sequelae of stroke, trauma, tumours or due to systemic diseases. In most cases, the pain often present in multiple sclerosis is of such origin. In recent years, interest has focussed on neuropathic pain induced by chemotherapy drugs (vincristine, paclitaxel, oxaliplatin, bortezomib, etc.)

The characteristics of this pain vary from patient to patient, but usually have ongoing burning or electric shock sensations; paresthesia is often present, i.e. abnormal sensations even in the areas surrounding the primary site of pain. These sensations are known as hyperalgesia, when a slightly painful stimulation in fact creates a very strong pain, and allodynia, when a non-painful stimulation, which can be simply stroking the skin or the weight of a sheet, is perceived as pain.

This type of pain does not respond well to the most common analgesics such as acetyl salicylic acid, paracetamol or the much used non-steroidal antiinflammatory drugs, and even morphine is only partially effective. This type of pain is difficult to cure and as yet no specific treatments exist; it is one of the most frustrating problems in analgesic therapy.

The most commonly used drugs to treat this type of pain are the anticonvulsants such as gabapentin, carbamazepine and lamotrigine, lidocaine in patch form (not yet available in Italy), tramadol, tricyclic antidepressants such as amitriptyline or the better tolerated nortriptyline. Tramadol and opioid drugs are to be used with particular care because of their dependence potential, and tricyclic antidepressants can have serious side effects particularly in the elderly, If good pain control is not achieved with a single first choice drug, a combination of several drugs is justified since the molecular mechanisms acted on by the various drug categories are different.

There is hence an evident need to provide molecules which are at least alternatives to those currently available, which are effective in controlling neuropathic pain and possibly present fewer side effects such as dependency or behavioural changes.

### Summary of the invention

The object of the present Invention are compounds of formula (I) 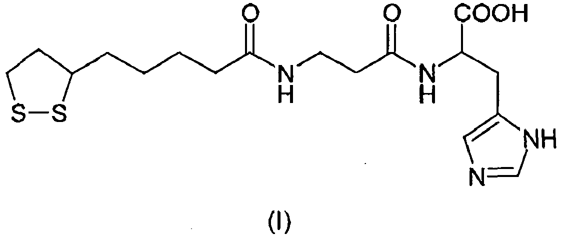 which are meant to include all possible optical isomers such as enantiomers and/or diastereoisomers, mixtures thereof, either as racemates or in various ratios, and Inorganic or organic salts (pharmaceutically acceptable).

Surprisingly the aforestated compounds have demonstrated an analgesic effect comparable to that observed for tramadol, pregabalin and ibuprofen. This effect was found to be statistically significant in *in vivo* experiments but at lower doses than the known drugs.

The compounds of the invention are therefore useful as medicaments, in particular as analgesics for treating neuropathic pain.

A further aspect of the invention is a process for preparing the aforestated compounds of formula (I) starting from lipoic acid and carnosine.

### Detailed description of the invention

Those compounds of formula (I) in which the stereogenic centre deriving from carnosine is in the L configuration are preferred.

Those compounds of formula (I) which are in the form of Na carboxylate are preferred.

In particular, a racemic mixture of the compounds of formula (I) in the form of sodium salt, in which the stereogenic centre deriving from carnosine is in the L configuration, was subjected to *in vivo* pharmacological tests which have enabled their surprising analgesic effects to be discovered. The aforestated racemic mixture, tested at doses of 10 and 30 mg kg⁻¹ per os, was found to be effective in reversing, in a statistically significant and dose-dependent manner, the hyperalgesia induced in the rat by loose ligation of the sciatic nerve. The demonstrated effect is greater, albeit slightly, than that shown by tramadol 100 mg kg⁻¹ per os and pregabalin 200 mg kg⁻¹ per os, in the same experimental model. At the same doses, the racemic mixture under study was also shown to be effective in reversing the pain threshold reduction induced by the intra-articular injection of monoiodide acetate (osteoarthritis model) and oxaliplatin (chemotherapeutic agent at high neurotoxic strength) but not the antiretroviral ddC.

The racemic mixture at the active doses does not change the behavioural parameters of the rat, such as: motor co-ordination, spontaneous movement and exploratory activity as assessed using the rotarod and hole-board tests. This confirms that the observed anti-hyperalgesic effects are not due to a change in the animal's behaviour.

In the hot plate test (mouse - acute thermal stimulus) the racemic mixture was also able to increase the pain threshold in a dose-dependent manner, achieving a high analgesic effectiveness; in the writhing test (mouse - acute chemical stimulus) the reduction in number of writhes is such as to be comparable to that obtained with ibuprofen at a dose of 100 mg kg ⁻¹ per os.

A second aspect of the invention relates to the compounds of formula (I) as aforedescribed for use as medicaments. This use is supported by the preliminary data obtained, and also by the good therapeutic index of the racemic mixture as an example of the aforedescribed compounds of formula (I). In particular the compounds of formula (I) as aforedescribed are useful, as analgesics, for treating neuropathic pain.

A further aspect of the invention relates to pharmaceutical compositions comprising at least one compound of formula (I) and at least one other pharmaceutically acceptable ingredient.

The compounds of formula (I) as aforedescribed can be preferably prepared by means of two synthetic steps in which firstly the lipoic acid is reacted with a reagent capable of activating the carboxylic acid group, followed by formation of an amide bond by the addition of carnosine.

Given the different lipo-hydrophilic characteristics of the two molecules, the choice of reaction conditions (solvent and accompanying agent) is not insignificant, and neither is the purification of the final product. Many of the conditions generally used for similar molecules and similar reactions have not in fact led to the required product in the desired yields and purity.

Preferably the lipoic acid is activated by treatment with N-hydroxysuccinimide to obtain the compounds of formula (II) 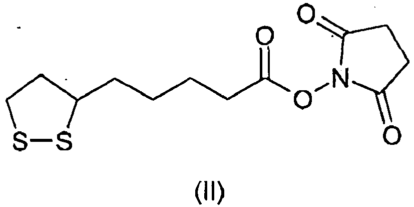 which include the two possible enantiomers and mixtures thereof.

The aforesaid compounds of formula (II) are isolable and are useful intermediates for the synthesis of the compounds of formula (I) as aforedescribed.

The compounds of formula (II) as aforedescribed can be obtained by reacting lipoic acid with N-hydroxysuccinimide in the presence of a carbodiimide (e.g. cyclohexyl carbodiimide, 4-(4,6-dimethoxy-1,3,5-triazin-2-yl)-4-methylmorpholinium chloride (DMTMM), O-(7-azabenzotrlazol-1-yl)-N,N,N',N'-tetramethyluronium hexafluorophosphate (HATU), oxalyl chloride, isopropenyl chloroformate (IPCF)) in a polar aprotic solvent (e.g. THF, DMF, diethyl ether, nitromethane, acetonitrile, triethylamine). Whereas adding the carbodiimide to the remaining reaction mixture is preferably carried out at a temperature of 0-5°C, the reaction mixture is then heated to ambient temperature (20-25°C) and left to react for a time sufficient to complete the reaction (e.g. 5-6 hours).

The compounds of formula (II) as aforedescribed can then be reacted with carnosine to obtain the compounds of formula (I) as aforedescribed.

Preferably the reaction between the Intermediates of formula (II) and carnosine is carried out in mixtures of H₂O/ polar aprotic solvent (e.g. DMSO, DMF, acetonitrile, nitromethane, THF) in the presence of a base (e.g. NaHCO₃, Na₂CO₃, triethylamine, pyridine, lutidine). Preferably the Intermediate of formula (II), dissolved in a polar aprotic solvent (e.g. DMF), is slowly added to the rest of the reaction mixture (in 0.5-1.5 hours) at a temperature between -5 and +5°C; the reaction mixture is then left at the above temperature for a time sufficient to complete the reaction (e.g. 3-6 hours).

The product obtained at the end of the process, once the solvent is evaporated, is a solid and can be conveniently purified by crystallization.

The compounds of formula (I), at the end of the aforedescribed process, are obtained in the form of salts in which the cation corresponds to that of the base used in the coupling reaction with carnosine.

The present invention can be better understood in the light of the following embodiments.

### Experimental part

The following describes an example of the synthesis of a compound of the invention in a racemic mixture starting from lipoic acid and L-carnosine by means of the steps shown in the following scheme:

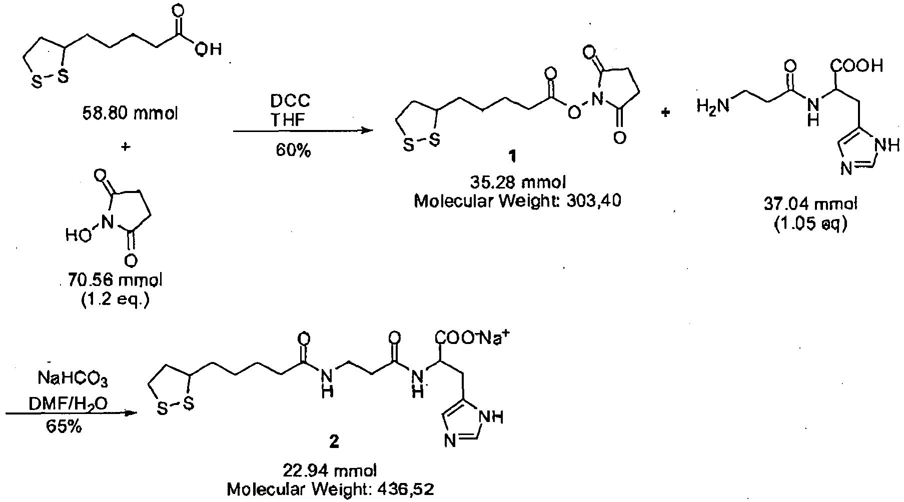

### Synthesis of compound 1

A solution of cyclohexylcarbodiimide (1.2 g, 5.82 mmol in 2 ml of THF) is added slowly to a solution of R/S lipoic acid (1 g, 4.85 mmol) and N-hydroxysuccinimide (674 mg, 5.82 mmol) in 30 ml of THF at 4°C. The solution is heated to ambient temperature (20-25°C) and agitation is maintained for 5.5 hours. The solid is removed by filtration and the organic solvent is evaporated to obtain a yellow solid which is purified by crystallization (ethyl acetate: hexane - 1:1). The pure compound 1 is hence obtained with a yield of 57%.
¹H NMR (CDCl₃): δ 1.4-2.1 (m), 2.4-2.6 (m), 2.7 (t), 2.9 (as), 3.1-3.3 (m), 3.5-3.7 (m).

### Synthesis of compound 2

The compound 1 (450 mg, 1.48 mmol) dissolved in DMF (3 ml) is added in 1 hour at 0°C to a solution of L-carnosine (335 mg, 1.48 mmol) and NaHCO₃ (124 mg, 1.48 mmol) in 6 ml of a 1:1 H₂O:DMSO mixture. After 4 hours the reaction is concluded. The DMF and H₂O are removed and the solid obtained is purified by crystallization (acetone), to hence obtain compound 2 as a sodium salt, pure by HPLC and with a yield of 85%.
MP > 213°C (dec)
¹H NMR (CD₃OD): δ 1.4-2.0 (m), 2.1-2.5 (m), 2.9-3.2 (m), 3.3-3.6 (m), 4.5 (dd), 6.8 (s), 7.6 (d).
¹³C NMR (CD₃OD): δ 25.4, 28.6, 29.2, 34.5, 35.6, 35.7, 38.0, 40.0, 54.8, 56.2, 118.8, 132.3, 134.2, 171.4, 174.4, 176.4.
ESI-MS: 437.45 (M+Na⁺); 459.45 (M-1+2N⁺).

### Pharmacological tests

The racemic mixture of the aforesaid (henceforth also identified as compound 2) was found to be able to reverse, in a statistically significant manner, the hyperalgesia induced in the rat by loose ligation of the sciatic nerve carried out 14 days earlier, even at a dose of 10 mg/kg per os. The effect, which began 15 minutes after administration, remained unchanged up to 45 minutes; for the same observation times, the same racemic mixture administered at a dose of 30 mg kg⁻¹ per os demonstrated a greater effectiveness. The observed effect is greater, albeit slightly, than that obtained by tramadol 100 mg kg ⁻¹ per os and pregabalin 200 mg.kg ⁻¹ per os, in the same experimental model.

### EFFECT OF COMPOUND 2 IN A RAT MODEL OF MONONEUROPATHY dx EVALUATED USING THE PAW PRESSURE TEST

| | | | *PAW PRESSURE (g)* | | |
|---|---|---|---|---|---|
| *TREATMENT* | *DOSE mg*/*kg per os* | *PAW* | *BEFORE TREATMENT* | *AFTER TREATMENT (15 min)* | |
| | CMC | left | 61.3 ± 3.7 | 60.8 ± 3.7 | |
| | CMC | right | 24.8 ± 3.3 | 25.9 ± 3.1 | |
| Compound 2 | 10 | l | 61.9 ± 3.4 | 69.5 ± 4.4 | |
| Compound 2 | 10 | r | 25.2 ± 3.3 | 48.3 ± 3.5* | |
| Compound 2 | 30 | l | 59.2 ± 3.8 | 81.6 ± 3.1* | |
| Compound 2 | 30 | r | 24.8 ± 3.2 | 66.3 ± 3.3* | |
| TRAMADOL | 100 | l | 58.3 ± 3.1 | 72.3 ± 3.0^ | |
| TRAMADOL | 100 | r | 25.7 ± 2.2 | 55.8 ± 2.5* | |
| PREGABALIN | 200 | l | 57.3 ± 3.5 | 70.4 ± 3.5 | |
| PREGABALIN | 200 | r | 26.3 ± 3.0 | 58.2 ± 3.1* | |
| | Rats per group; *P< 0.01 | | | | |

At the same doses of 10 and 30 mg kg ⁻¹ per os, the racemic mixture under study was also found to be effective in reversing the pain threshold reduction induced by the intra-articular injection of monoiodide acetate at a dose of 2 mg in a 25 µl volume (osteoarthritis model). In this case the effect is statistically significant up to 45 minutes after administration at the lower dose and up to 60 minutes at the three-fold dose.

### EFFECT OF COMPOUND 2 ON OSTEOARTHRITIS INDUCED PAIN EVALUATED IN THE RAT BY THE PAW PRESSURE TEST

| | PAW PRESSURE (g) | | | |
|---|---|---|---|---|
| TREATMENT | DOSE mg/kg p. o. | AFTER TREATMENT | | |
| | | Pre-test | 15 min | 30 min |
| | | | | |
| | CMC | 61.5 ± 3.0 | 58.3 ± 2.4 | 62.7 ± 3.7 |
| | CMC | 23.8 ± 2.7 | 21.5 ± 3.0 | 24.5 ± 3.0 |
| | | | | |
| Compound 2 | 10 | 24.5 ± 2.1 | 49.3 ± 2.5* | 45.3 ± 3.9* |
| Compound 2 | 30 | 24.4 ± 2.5 | 59.1 ± 3.3* | 54.7 ± 3.6* |

| | | | | |
|---|---|---|---|---|
| Intra-articular treatment: Monosodium iodoacetate (MIA) 2 mg in a 25 ml volume was injected Intra-articularly into the anaesthetized rat Each value represents the mean of 4 rats ^P<0.05; *P< 0.01 compared with rats treated with MIA/CMC. Fernihough J. et al. Pain 112: 83-93 (2004). | | | | |

In the case of hyperalgesia induced by administration of the chemotherapeutic agent oxaliplatin injected for 5 days in the week at a dose of 2.4 mg kg ⁻¹ i.p. for 3 weeks, the profile exhibited by the racemic mixture is very similar to that noted in the preceding osteoarthritis model, only that the duration of the effect is more brief.

### EFFECT OF COMPOUND 2 ON OXALIPLATIN-INDUCED PAIN IN THE RAT PAW PRESSURE TEST OF COMPOUND 2 ON OXALIPLATIN INDUCED HYPERALGESIA

| | EVALUATED BY THE PAW PRESSURE TEST (g) | | | | | | |
|---|---|---|---|---|---|---|---|
| TREATMENT no. 1 | TREATMENT no. 1 | DOSE per os mg/kg | AFTER TREATMENT | | | | |
| | | | Pre-test | 15 min | 30 min | 45 min | 60 min |
| SALINE | CMC | | 60.6 ± 2.5 | 61.8 ± 3.4 | 58.4 ± 3.1 | 61.0 ± 3.0 | 57.9 ± 3.6 |
| OXALIPLATIN | CMC | | 24.7 ± 33 | 23.4 ± 2.5 | 25.6 ± 3.7 | 24.8 ± 3.1 | 24.2 ± 2.7 |
| OXALIPLATIN | COMPOUND 2 | 10 | 23.8 ± 3.6 | 34.6 ± 3.1^ | 36.7 ± 3.5^ | 31.9 ± 3.5 | 26.9 ± 2.8 |
| OXALIPLATIN | COMPOUND 2 | 30 | 22.9 ± 3.5 | 43.6 ± 3.9* | 48.9 ± 3.6* | 41.6 ± 3.6* | 31.7 ± 3.3 |

| | | | | | | | |
|---|---|---|---|---|---|---|---|
| ^P< 0.05 versus rats treated with oxaliplatin/CMC | | | | | | | |

The racemic mixture under study, at a dose of 30 mg kg ⁻¹ per os does not alter the behavioural parameters of the mouse, such as: motor coordination, spontaneous motility and exploratory activity as evaluated using the rotarod and hole-board tests. In particular, in the case of the rotarod, the number of falls from the rotating rod progressively decreases with repeated falls. This confirms that the observed anti-hyperalgesic effects are not due to a change in the animal's behaviour.

### ROTAROD TEST

| **FALLS IN 30 SEC** | | | | |
|---|---|---|---|---|
| | Pre-test | *15 min* | *30 min* | *45 min* |
| | | | | |
| CMC | 5.3 ± 0.5 | 3.8 ± 0.4 | 1.7 ± 0.4 | 1.1 ± 0.3 |
| COMPOUND 2 30 mg/kg⁻¹ per os | 5.2 ± 0.3 | 3.9 ± 0.3 | 1.3 ± 0.3 | 0.8 ± 0.2 |

| | | | | |
|---|---|---|---|---|
| Each value represents the mean of 10 mice. | | | | |

### HOLE-BOARD TEST

| | No. of movements | ***No. of inspections*** |
|---|---|---|
| CMC | 31.5 ± 8.2 | 41.6 ± 8.9 |
| COMPOUND2 30 mg/kg⁻¹ per os | 30.3 ± 7.7 | 37.8 ± 7.5 |

| | | |
|---|---|---|
| Each value represents the mean of 10 mice. | | |

In the hot plate test (mouse - acute thermal stimulus) the racemic mixture was also able to raise the pain threshold in a dose-dependent manner for the dose range comprised between 1 and 30 mg kg ⁻¹ per os, achieving a high analgesic effect.

### EFFECT OF COMPOUND 2 ON MICE USING THE HOT PLATE TEST

| Treatment | no. of mice | Pre-test | 15 min | 30 min | 45 min | 60 min |
|---|---|---|---|---|---|---|
| CMC | 5 | 16.0±1.4 | 15.0±1.3 | 14.9±0.9 | 15.8±1.4 | 15.0±0.8 |
| per os | | | | | | |
| COMPOUND 2 | 7 | 15.7±0.8 | 18.7±2.7 | 25.1±2.7* | 14.0±2.0 | 15.7±2.6 |
| 1 mg/kg per os | | | | | | |
| COMPOUND 2 | 7 | 15.3±0.9 | 25.9±2.8* | 22.3±2.6* | 19.3±1.9* | 17.4±0.8 |
| 10 mg/kg per os | | | | | | |
| COMPOUND2 | 5 | 15.7±1.3 | 29.7±2.8* | 26.0±2.7* | 20.3±2.9* | 18.7±2.1 |
| 30 mg/kg per os | | | | | | |

| | | | | | | |
|---|---|---|---|---|---|---|
| ^P< 0.05; *P<0.01 versus the mouse group treated with CMC. | | | | | | |

In the writhing test (mouse - acute chemical stimulus) the reduction in number of writhes at the 30 mg kg ⁻¹ per os dose is such as to be comparable to that obtained with ibuprofen at a dose of 100 mg kg-1 per os. The 10 mg kg ⁻¹ per os dose was also found to be effective. In this test, analgesia induced by the racemic mixture at a dose of 30 mg kg ⁻¹ per os was not antagonized by the opioid antagonist naloxone (1 mg kg ⁻¹ i. p.), thus demonstrating that the observed effect was not morphine-like.

| **EFFECT OF COMPOUND 2 IN MICE USING ABDOMINAL WRITHING TEST (ACETIC ACID 0.6%)** | | | |
|---|---|---|---|
| *TREATMENT per os* | *no. of mice* | *Dose per os mg kg⁻¹* | *no. of writhes* |
| CMC | 8 | | 32.4± 2.9 |
| | | | |
| COMPOUND 2 | 8 | 1 | 28.5± 2.8 |
| COMPOUND 2 | 8 | 10 | 21.8± 3.1* |
| COMPOUND 2 | 8 | 30 | 12.7± 2.6* |
| | | | |
| IBUPROFEN | 8 | 30 | 20.3± 2.2* |
| IBUPROFEN | 8 | 100 | 13.5± 2.0* |
| | | | |
| NALOXONE 1 + EDR 08-2111 30 | 8 | | 13.6± 3.0* |
| Compound-2 and IBUPROFEN were administered per os 30 min before the test. *P <0.01 versus the group treated with carrier. | | | |
| Naloxone 1 mg kg⁻¹ i.p. 15 min before compound 2 | | | |

## Claims

1. Compounds of formula (I): 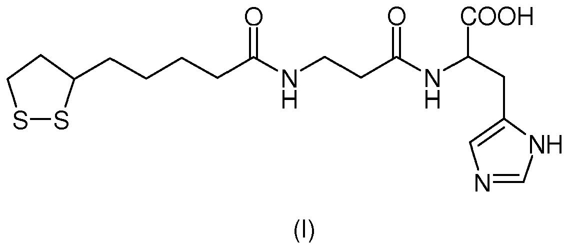 which are meant to include all possible optical isomers such as enantiomers and/or diastereoisomers, mixtures thereof, either as racemates or in various ratios, and inorganic or organic salts.

2. Compounds of formula (I) according to claim 1 for use as a medicament.

3. Compounds for use according to claim 2 as analgesics.

4. Compounds of formula (I) for use according to claim 3 in the treatment of neuropathic pain.

5. Compositions comprising at least one compound of formula (I) according to claim 1 and at least one other pharmaceutically acceptable ingredient.

6. Compounds of formula (II) 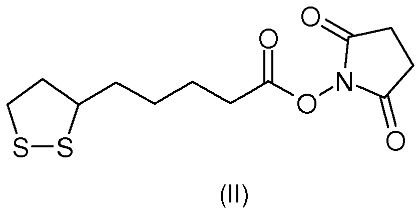 which is meant to include the two possible enantiomers and mixtures thereof.

7. Use of a compound of formula (II) according to claim 6 as intermediate in the preparation of a compound of formula (I) according to claim 1.

8. Process for the preparation of compounds of formula (I) according to claim 1 starting from lipoic acid and carnosine.

9. Process according to claim 8 wherein an intermediate of formula (II) according to claim 6 is used.

## Patentansprüche

1. Verbindungen der Formel (I) 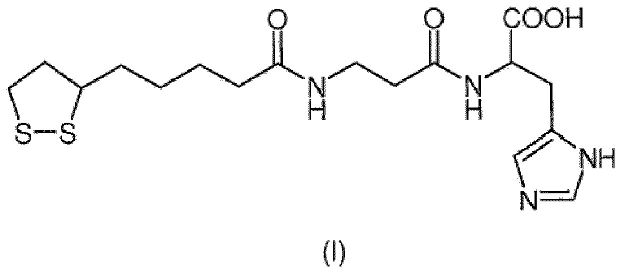 bei denen verstanden wird, daß sie alle möglichen optischen Isomere wie Enantiomere und/oder Diastereoisomere, Mischungen davon, entweder als Racemate oder in verschiedenen Verhältnissen, und anorganische und organische Salze einschließen.

2. Verbindungen der Formel (I) nach Anspruch 1 zur Verwendung als Medikament.

3. Verbindungen zur Verwendung nach Anspruch 2 als Analgetika.

4. Verbindungen der Formel (I) zur Verwendung nach Anspruch 3 bei der Behandlung von neuropathischen Schmerzen.

5. Zubereitungen, umfassend wenigstens eine Verbindung der Formel (I) nach Anspruch 1 und wenigstens einen anderen pharmazeutisch annehmbaren Inhaltsstoff.

6. Verbindungen der Formel (II) 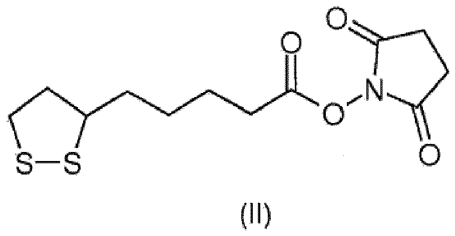 bei denen verstanden wird, daß sie die zwei möglichen Enantiomere und Mischungen davon einschließen.

7. Verwendung einer Verbindung der Formel (II) nach Anspruch 6 als Zwischenstufe beim Herstellen einer Verbindung der Formel (I) nach Anspruch 1.

8. Verfahren für das Herstellen von Verbindungen der Formel (I) nach Anspruch 1, ausgehend von Liponsäure und Carnosin.

9. Verfahren nach Anspruch 8, worin eine Zwischenstufe von Formel (II) nach Anspruch 6 verwendet wird.

## Revendications

1. Composés de formule (I) : 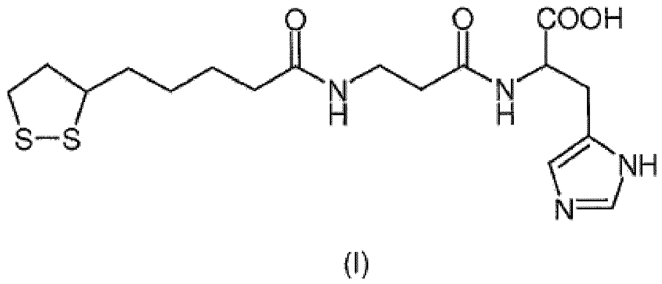 qui sont censés inclure tous les isomères optiques possibles tels que des énantiomères et/ou des diastéréo-isomères, des mélanges de ceux-ci, soit sous la forme de racémates soit dans des rapports divers, et des sels inorganiques ou organiques.

2. Composés de formule (I) selon la revendication 1 pour une utilisation en tant que médicament.

3. Composés pour une utilisation selon la revendication 2 en tant qu'analgésiques.

4. Composés de formule (I) pour une utilisation selon la revendication 3 dans le traitement de la douleur neuropathique.

5. Compositions comprenant au moins un composé de formule (I) selon la revendication 1 et au moins un autre composant pharmaceutiquement acceptable.

6. Composés de formule (II) 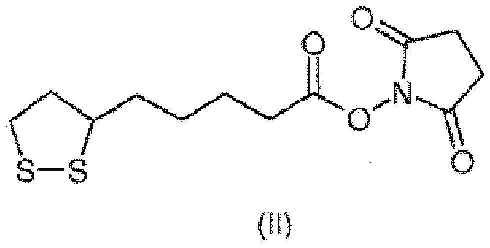 qui sont censés inclure les deux énantiomères possibles et des mélanges de ceux-ci.

7. Utilisation d'un composé de formule (II) selon la revendication 6 en tant qu'intermédiaire dans la préparation d'un composé de formule (I) selon la revendication 1.

8. Procédé de préparation des composés de formule (I) selon la revendication 1 en démarrant à partir d'acide lipoïque et de carnosine.

9. Procédé selon la revendication 8, dans lequel un intermédiaire de formule (II) selon la revendication 6 est utilisé.
